# EUROPEAN PATENT SPECIFICATION

(11) **EP 3 122 902 B1**
(45) Date of publication and mention of the grant of the patent: **16.05.2018**
(21) Application number: 15717786.6
(22) Date of filing: 27.03.2015
(51) Int. Cl.: C12Q 1/68

(54) **METHOD OF PREDICTING THE TUMOR RESPONSE TO DNA METHYLATION INHIBITORS**
VERFAHREN ZUR VORHERSAGE DER TUMORREAKTION AUF INHIBITOREN DER DNA-METHYLIERUNG
PROCÉDÉ DE PRÉDICTION DE LA RÉPONSE DE TUMEURS À DES INHIBITEURS DE LA MÉTHYLATION D'ADN

(30) Priority: 27.03.2014 EP 14161897
(43) Date of publication of application: 01.02.2017
(73) Proprietor: Palacký University In Olomouc, 771 47 Olomouc (CZ)
(72) Inventor: AGRAWAL, Khushboo, 779 00 Olomouc (CZ); DZUBAK, Petr, 751 03 Brodek u Prerova (CZ); FRYDRYCH, Ivo, 798 14 Olsany u Prostejova 505 (CZ); HAJDUCH, Marian, 793 05 Moravsky Beroun (CZ)
(74) Representative: Hartvichova, Katerina
(86) International application number: PCT/CZ2015/000029
(87) International publication number: WO 2015/144102

(56) References cited:
- US-A1- 2011 301 110
- ROMAIN AUCAGNE ET AL: "Transcription intermediary factor 1 gamma is a tumor suppressor in mouse and human chronic myelomonocytic leukemia", JOURNAL OF CLINICAL INVESTIGATION, AMERICAN SOCIETY FOR CLINICAL INVESTIGATION, US, vol. 121, no. 6, 1 June 2011 (2011-06-01), pages 2361-2370, XP002684885, ISSN: 0021-9738, DOI: 10.1172/JCI45213 [retrieved on 2011-05-02]
- T. BRAUN ET AL: "Molecular predictors of response to decitabine in advanced chronic myelomonocytic leukemia: a phase 2 trial", BLOOD, vol. 118, no. 14, 6 October 2011 (2011-10-06), pages 3824-3831, XP055059701, ISSN: 0006-4971, DOI: 10.1182/blood-2011-05-352039
- I. KHAN ET AL: "New Strategies in Acute Myeloid Leukemia: Redefining Prognostic Markers to Guide Therapy", CLINICAL CANCER RESEARCH, vol. 18, no. 19, 14 August 2012 (2012-08-14), pages 5163-5171, XP055198700, ISSN: 1078-0432, DOI: 10.1158/1078-0432.CCR-12-0313
- Johannes Zuber ET AL: "RNAi screen identifies Brd4 as a therapeutic target in acute myeloid leukaemia", Nature, 27 October 2011 (2011-10-27), pages 524-528, XP055130770, England DOI: 10.1038/nature10334 Retrieved from the Internet: URL:http://search.proquest.com/docview/907 242428
- SHI JUNWEI ET AL: "The Mechanisms behind the Therapeutic Activity of BET Bromodomain Inhibition", MOLECULAR CELL, vol. 54, no. 5, 5 June 2014 (2014-06-05), pages 728-736, XP028849507, ISSN: 1097-2765, DOI: 10.1016/J.MOLCEL.2014.05.016

## Description

### Field of Art

The invention is directed to a method for predicting the tumor response (i.e. sensitive or resistant) towards DNA methylation inhibitors.

### Background Art

Resistance to chemotherapeutic treatment is one of the major impediments forefending the successful cancer therapy (Gottesman M.M. et al., Nature Reviews Cancer 2002; 2, 48-58). Although the research has unraveled the main molecular signatures of resistance to chemotherapy, including intracellular inactivation of the drug (Garattini S. et al., European Journal of Cancer 2007; 43, 271-82), defects in DNA mismatch repair (Fink D. et al., Clinical Cancer Research 1998; 4, 1-6), evasion of apoptosis (Hanahan D. et al., Cell 2000; 100, 57-70), membrane transporters (Huang Y. et al., Cancer Research 2004; 64, 4294-301) and many more, the failure of cancer chemotherapy remains frequently unresolved. Moreover, a particular drug resistance mechanism defined in cell culture systems and animal models does not necessarily correlate with the individual molecular pathology in clinic (Cimoli G. et al., Biochimica et Biophysica Acta 2004; 1705, 103-20). This has underlined the paramount importance for investigating the additional targets to sensitize the cancer patients, resistant to a particular drug, and tailor the alternative therapeutic regimens for individual patients. Currently, epigenetics has emerged as one of the most promising fields expanding the boundaries of oncology and aberrant DNA methylation remains the consistent hallmark due to its frequent involvement in all types of cancer (Rodríguez-Paredes M. et al., Nature Medicine 2011; 17, 330-39). Cytosine analogues, 5-azacytidine (AZA) and 2'-deoxy-5-azacytidine (DAC) are currently one of the most effective epigenetic drugs (Stresemann C. et al., International Journal of Cancer 2008; 123, 8-13), which function by inhibiting the expression of *de novo* DNA methyltransferases, and have shown substantial potency in reactivating tumor suppressor genes silenced by aberrant DNA methylation (Karahoca M. et al., Clinical Epigenetics 2013; 5, 3). The prototypical DNA methyltransferase inhibitors, AZA and DAC are one of the few drugs that patients suffering from myelodysplastic syndromes (MDS) and acute myeloid leukemia (AML) respond to, and have been approved by the Food And Drug Administration (FDA) and European Medicines Agency (EMA) for the treatment of MDS (Saba H.I. et al., Therapeutics and Clinical Risk Management 2007; 3, 807-17). Apart from being established therapies for myeloid malignancies, they seemed promising in eradicating solid tumors during early clinical trials (Cowan L.A. et al., Epigenomics 2010; 2, 71-86). However, like other anticancer drugs, resistance to these hypomethylating agents is a major barrier reversing the effective epigenetic therapy. Most patients do not respond to therapy and experience primary resistance whereas those responding initially acquire secondary resistance and succumb to the disease, despite of continued therapy (Prébet T. et al., Journal of Clinical Oncology 2011; 29, 3322-7). Molecular mechanisms elucidating the cause of resistance to these drugs *in vitro* are diverse, including insufficient drug influx by membrane transporters, deficiency of the enzyme deoxycytidine kinase required for drug activation, or deamination by cytidine deaminase leading to increased drug metabolism, but they fail to explain acquired resistance in patients. In addition, it has also been implemented that secondary resistance to DAC is likely to be independent of DNA methylation and resistance develops regardless of persistent demethylation (Qin T. et al., PLOS ONE 2011; 6, e23372). Also, it is undeniable fact that re-expression of epigenetically silenced tumor suppressor genes following DAC treatment is transitory (Kagey J.D. et al., Molecular Cancer Research 2010; 8, 1048-59). Withdrawal of DAC eventually results in gene re-silencing leading to resistance whereas sustained gene re-expression concords with the clinical response, supporting the role of gene re-silencing in development of drug resistance (Hesson L.B. et al., PLOS Genetics 2013; 9, e1003636). If the focus is laid on gene re-silencing as the prerequisite for resistance, it highlights the central dogma of epigenetics which articulates that the gene silencing mechanisms (DNA hypermethylation, mutations in chromatin remodeling complexes and multiple post-translational histone modifications) are not isolated from each other but interlinked (Grant S. et al., Clinical Cancer Research 2009; 15, 7111-3). In this context, bromodomains (BRDs), chromatin effector modules that recognize and bind to ε-N-acetyl lysine motifs have rapidly emerged as exciting new targets in the quest for clinical progress in cancer (Muller S. et al., Expert Reviews in Molecular Medicine). The role of multiple bromodomain genes in restricting the spread of heterochromatic silencing has been explored in the past (Jambunathan N. et al., Genetics 2005; 171, 913-22). In addition, bromodomain proteins play a critical role in gene activation by recruitment of the factors necessary for transcription (Josling G.A. et al., Genes 2012; 3, 320-43).

The present invention exposes such bromodomain containing genes and/or proteins coded by the gene, the expression of which was differentially regulated during the development of resistance, and targeting of which may sensitize the patients suffering from resistance towards DNA methylation inhibitors. Therefore, the present invention provides a method for determining the response of the patients (i.e. sensitive or resistant) towards DNA methylation inhibitors.

### Disclosure of the invention

The first embodiment of the invention is a method for predicting the sensitivity of a patient suffering from a cancer disease to DNA methylation inhibitor therapy, which comprises determining *in vitro* in the cancer cells taken from the patient and comparing with values for parent type of cells
- the level of expression of BRD4 gene, wherein the decrease in expression determines resistance, optionally in combination with one or more or all of the further genes selected from the group comprising:

| **Gene** | **Change in expression determining resistance** |
|---|---|
| ASH1L | increase |
| ATAD2 | decrease |
| BAZ1B | decrease |
| BAZ2A | increase |
| BAZ2B | decrease |
| BRD1 | decrease |
| BRD2 | increase |
| BRD3 | increase |
| BRD7 | decrease |
| BRD8 | decrease |
| BRWD1 | increase |
| CECR2 | increase |
| CREBBP | increase |
| EP300 | increase |
| KAT2A | increase |
| KAT2B | increase |
| KMT2A | increase |
| SMARCA2 | increase |
| SP100 | increase |
| SP110 | increase |
| TRIM66 | decrease |
| ZMYND8 | decrease |
| ZMYND11 | decrease |

and subsequently determining the resistance or sensitivity of the patient towards the said treatment based on the information provided above.

The change in the level of expression (up-regulation or down-regulation) of the genes and/or proteins coded by the genes, listed in the relevant table was observed repeatedly in several drug resistant cell lines in comparison with their genetically identical drug sensitive counterpart, and is therefore the indicator of resistance towards DNA methylation inhibitor, 2'-deoxy-5-azacytidine (DAC).
Preferably, the level of expression of a combination of BRD4 with at least two, three, four, five, six, seven, eight, nine or ten bromodomain containing genes is determined. Most preferably, the level of expression of all herein listed bromodomain containing genes and/or the level of expression of all herein listed bromodomain containing proteins is determined.

The method of determination of resistance is particularly useful in cancers selected from carcinomas, sarcomas, melanomas, lymphomas, and leukemia.

### Brief description of Drawings

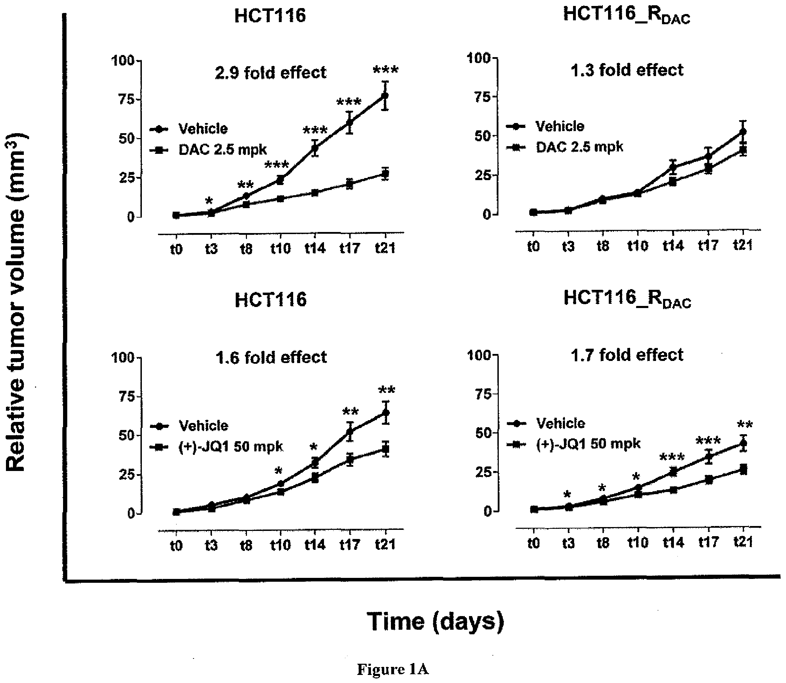

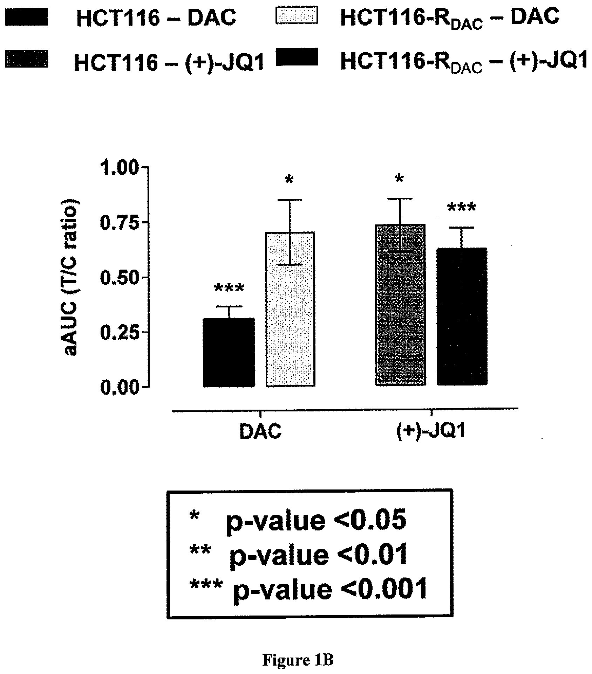

Figure 1: Anti-tumor activity of DAC and (+)-JQ1. Decrease in fold effect and less significant T/C ratio for DAC clearly indicates towards resistance of HCT116-R_{DAC} (fold effect 1.3, p < 0.05) in comparison with parental HCT116 (fold effect 2.9, p < 0.001), contradictorily, increase in fold effect for (+)-JQ1 indicates higher sensitivity of HCT116-R_{DAC} (fold effect 1.7, p < 0.001) in comparison with parental HCT116 (fold effect 1.6, p < 0.05).

### Examples of carrying out the invention

### Cell culture

To study the mechanism of resistance towards DNA methylation inhibitor, 2'-deoxy-5-azacytidine, we used the human colorectal cancer cell line (HCT116), human promyelocytic leukemia cells (HL-60), and human breast adenocarcinoma cell line (MCF-7) obtained from American Type Culture Collection (Manassas, VA). The cell lines were cultured in complete growth media (Sigma-Aldrich, St. Louis, MO), supplemented with fetal bovine serum (FBS, PAN-Biotech GmbH, Aidenbach, Germany), 100 U/mL penicillin (Biotika, Slovenská L'upča, Slovak Republic) and 50 µg/mL streptomycin (Sigma-Aldrich), and the cultures were maintained at 37°C and 5% CO₂, in a humidified incubator. The cell line, HCT116 was grown in McCoy's 5A medium supplemented with 10% FBS and 3 mM L-glutamine (Sigma-Aldrich), HL-60 was grown in Iscove's Modified Dulbecco's Medium with 20% FBS, and MCF-7 was grown in RPMI-1640 medium with 10% FBS.

### Development of resistant cell lines

The DAC resistant HCT116 cell lines were developed using two methods. Adaptation method: the cells were initially treated with 1x IC₅₀ concentration of the drug (0.28 µM; 5 days MTT test) which was gradually increased with the adaptation of resistance up to 10x IC₅₀ in subsequent passages. Rapid selection method: the cells were directly exposed to 5x IC₅₀ concentration of the drug which was further doubled to 10x IC₅₀. After long term exposure of the cells to cytotoxic dose of the drug, the bulk population was determined to be resistant. Cloning of the resistant cell population resulted in six resistant cell lines, R1.1, R1.2, R1.3, R1.4 (isolated by adaptation method) and R2.1, R2.2 (isolated by rapid selection method).

For cross validation of data obtained from comparative studies of HCT116 parental and resistant cells, we further developed DAC resistant MCF-7 and HL-60 cell lines. Resistant MCF-7 cells were developed using adaptation method, where cells were treated with 0.5 µM DAC which was gradually increased up to 5 µM with adaptation of resistance. However, DAC resistant HL-60 cells were obtained as a gift from the author (Qin T. et al., Blood 2009; 113, 659-67) and was further selected in our laboratory by treatment with 5 µM DAC.

Resistance to DAC was confirmed by the MTT-based cell survival assay and resistance index was calculated as the fold increase in the IC₅₀ of the resistant cell lines compared with the untreated control. All of the resistant cell lines were >100 µM resistant to DAC.

### RNA sequencing (RNA-Seq) based transcriptomics

RNA sequencing (RNA-Seq) utilizing high-throughput sequencing platforms have emerged as a powerful method for discovering, profiling and quantifying transcriptome by facilitating relatively unbiased measurements of transcript and gene expressions, ability to measure exon and allele specific expressions, and to detect the transcription of unannotated exons leading to identification of rare and novel transcripts (Pickrell J.K. et al., Nature 2010; 464, 768-72).

### Sample preparation/construction of cDNA library:

HCT116 parental and each of the six resistant cell lines were grown in petridishes with coverage greater than 80%. Cells were homogenized using 1 mL of TRI (trizol) reagent per 10 cm² of the monolayer culture and incubated at room temperature for 5 min, to allow the complete dissociation of nucleoprotein complexes. The homogenates were transferred to 1.5 mL microcentrifuge tubes and the total RNA was extracted by organic extraction method according to manufacturer's protocol (Ambion RiboPure Kit, Life Technologies, Carlsbad, CA). The integrity of the obtained RNA samples was analyzed (Agilent RNA 6000 Nano Kit, Agilent Technologies, Santa Clara, CA) using Agilent 2100 Bioanalyzer. 0.1-4 µg of the total RNA was used and the cDNA library was constructed according to manufacturer's protocol (TruSeq Stranded mRNA Sample Prep Kit, Illumina, San Diego, CA). Briefly, the poly-A containing mRNA molecules were purified using poly-T oligo attached magnetic beads in two rounds of purification which included RNA fragmentation and priming with random hexamers. The cleaved RNA fragments were reverse transcribed (RevertAid H Minus Reverse Transcriptase, Thermo Scientific, Waltham, MA) into first strand cDNA followed by second strand cDNA synthesis. The cDNA synthesis was complemented with an "End Repair" control at -20°C. A single 'A' nucleotide was added to 3' ends of the blunt fragments followed by the ligation of multiple indexing adaptors. The DNA fragments with adapter molecules on both ends were selectively enriched and amplified by PCR. The cDNA library thus prepared was validated and quantified (Agilent High Sensitivity DNA Kit, Agilent Technologies) using Agilent 2100 Bioanalyzer. Finally, the samples with different indexes were pooled together for sequencing.

### RNA Sequencing, Alignment and Variant Calling:

Transcriptome was sequenced by massively parallel signature sequencing (MPSS) using Illumina's ultra-high-throughput sequencing system, HiSeq 2500. The reads generated from the RNA Seq experiment were aligned to annotated human reference genome (HG₁₉) using Tophat 2 (Trapnell C. et al., Bioinformatics 2009; 25, 1105-11) and those aligning to exons, genes and splice junctions were counted. Tolerance was set to allow the maximum of two mismatches during an alignment and the reads aligning to multiple genomic locations were discounted. Variants (cSNPs, indels and splice junctions) were called after alignment by SAMtools (Li H. et al., Bioinformatics 2009; 25, 2078-79) and annotated by ANNOVAR (Wang K. et al., Nucleic Acids Research 2010; 38, e164). For the quantification of gene and transcript level expression, HTSeq package (Python) was used and differential expressions were reported after data normalization and statistical evaluation using DESeq package (R library). Statistical significance was determined by the binomial test and threshold for significance was set to 0.01.

### Mass spectrometry based proteomics

While transcriptomics studies provide insight into the roles of RNA and gene expression, it is ultimately the change in the level of protein expressions which affects the biological functions. Stable isotope labelling of amino acids in cell culture (SILAC) coupled with mass spectrometry had evolved as an invaluable tool in identification and development of novel biomarkers, by facilitating the quantification of differential protein levels in normal and pathophysiological states (Mann M., Nature Reviews Molecular Cell Biology 2006; 7, 952-58).

### SILAC/Preparation of lysates:

The parental cell line, HCT116 was cultured in SILAC medium (Thermo Scientific) substituted with heavy Lys-¹³C₆ and Arg-¹³C₆ and dialyzed FBS (Sigma-Aldrich) for about 8 doublings to reach the complete labelling. The labelled parental cell line was then mixed with each of the non-labeled resistant cell lines in 1:1 ratio. Cell mixture thus prepared was washed twice with ice cold 1x PBS with inhibitors [phosphatase inhibitors (5 mM sodium pyrophosphate, 1 mM sodium orthovanadate, 5 mM sodium fluoride), protease inhibitors (1 mM phenylmethylsulfonyl fluoride, Protease Inhibitor Cocktail; Sigma-Aldrich)] followed by a wash with 1x PBS without inhibitors, after which the cells were lysed using 200 µL of ice cold SILAC lysis buffer (20 mM Tris-HCl, 7 M Urea, 10 mM DTT, 1% Triton X-100, 0.5% SDS) per 2 x 10⁷ cells. Lysates were then sonicated using Branson Digital Sonifier and clarified by centrifugation at 14,000 rpm for 10 min and cleared supernatants were stored at -80°C.

### Fractionation/Enzymatic in-gel digestion:

Cell lysates (100 µL) were fractionated by molecular weight on 12% LDS-Tris-Glycine gel through a cylindrical gel matrix by continuous-elution gel electrophoresis (Mini Prep Cell, Bio-Rad, Hercules, CA) at constant power of 1 W for 3-4 hours. After electrophoresis, the gel was expelled from the tube and fixed (10% acetic acid, 35% ethanol), followed by rinsing with Milli-Q H₂O. Using a clean scalpel, the 90 mm gel piece was excised into 20 slices (∼4.5 mm each) which were further diced into small pieces (∼1 mm³) and transferred to 1.5 mL microcentrifuge tubes. The gel pieces were dehydrated with ACN (acetonitrile) by sonication for 5 min, followed by reduction with 50 mM tris-(2-carboxyethyl)phosphine at 90°C for 10 min. The reduced gel was dehydrated again, followed by alkylation with freshly prepared 50 mM IAA (iodoacetamide) for 60 min in dark. After alkylation, the gel was dehydrated twice with changes of ACN and H₂O (to ensure the complete removal of IAA), followed by dehydration with 50% ACN at last. Finally, the gel was subjected to enzymatic digestion by overnight incubation at 37°C with trypsin buffer (Trypsin Gold, Promega, Madison, WI) prepared according to manufacturer's protocol. After in-gel digestion of proteins, the peptide mixtures were extracted by dehydrating the gel [0.1% TFA (trifluoroacetic acid), 80% ACN] followed by rehydration (0.1% TFA) and dehydration with 50% ACN at last. The extraction buffer was concentrated until complete evaporation and the peptide mixtures were re-suspended (5 µL 80% ACN, 0.1% TFA) and diluted (145 µL 0.1% TFA). 150 µL of the peptide samples were loaded onto the column (MacroTrap, MICHROM Bioresources Inc., Auburn, CA) and desalted (0.1% TFA), followed by elution (0.1% TFA, 80% ACN). After purification, the elution buffer was completely evaporated and the purified peptides were re-suspended in mobile phase A [5% ACN, 0.1% FA (Formic acid)] for LC-MS analysis.

### LC-MS/MS:

20 µL of peptide samples in mobile phase A were loaded onto the trap column (Acclaim PepMap100 C18, 3 µm, 100 Å, 75 µm i.d. × 2 cm, nanoViper, Thermo Scientific) in UltiMate 3000 RSLCnano system (Thermo Scientific) for pre-concentration and desalting, at the flow rate of 5 µL/min. The trap column in turn was directly connected with the separation column (PepMap C18, 3 µm, 100 Å, 75 µm i.d. × 15 cm, Thermo Scientific) in EASY-Spray (Thermo Scientific), and the peptides separated by reverse-phase chromatography were eluted with 100 min linear gradient from 5 to 35% mobile phase B (80% ACN, 0.1% FA), at the flow rate of 300 nL/min and 35°C column temperature. After the gradient, the column was washed with mobile phase B and re-equilibrated with mobile phase A. For the acquisition of mass spectra, high performance liquid chromatography was coupled to an Orbitrap Elite Mass Spectrometer (Thermo Scientific) and the spectra were acquired in a data dependent manner with an automatic switch between MS and MS/MS scans using a top 20 method. MS spectra were acquired using Orbitrap analyzer with a mass range of 300-1700 Da and a target value of 10⁶ ions whereas MS/MS spectra were acquired using ion trap analyzer and a target value of 10⁴ ions. Peptide fragmentation was performed using CID method and ion selection threshold was set to 1000 counts.

### Data analysis:

Raw MS files were analyzed by MaxQuant version 1.4.1.3 (Cox J. et al., Nature Biotechnology 2008; 26, 1367-72) and MS/MS spectra was searched using Andromeda search engine (Cox J. et al., Journal of Proteome Research 2011; 10, 1794-1805) against the UniprotKB / Swiss-Prot-human database (generated from version 2013_09) containing forward and reverse sequences. The additional database of 248 common contaminants was included during the search (Geiger T. et al., Molecular & Cellular Proteomics 2012; 11, M111.014050). Mass calibration was done using the results from the initial search with a precursor mass tolerance of 20 ppm, however, in main Andromeda search, the precursor mass and the fragment mass was set to the tolerance of 7 ppm and 20 ppm respectively. The fixed modification of carbamidomethyl cysteine and the variable modifications of methionine oxidation and N-terminal acetylation were included for database searching. SILAC labels, R6 and K6 were used for the analysis of SILAC data. The search was based on enzymatic cleavage rule of Trypsin/P and a maximum of two miscleavages were allowed. The minimal peptide length was set to six amino acids and at least one unique peptide was must for protein identification. The false discovery rate (FDR) for the identification of peptide and protein was set to 0.01.

Bioinformatic analysis was performed with Perseus version 1.4.1.3. Filtrations were done to eradicate the identifications from databases of the reverse sequence and the common contaminants and to exclude proteins with < 3 valid values (only peptides quantified in three measurements were considered). The categorical annotation was supplied in the form of Gene Ontology (GO) biological process, molecular function and cellular component. For the quantification of differential expression, the data was transformed to log 2 and normalized by subtracting the median from each column. The fold change was calculated as mean of three values and significance was determined by calculating the p-value with a Benjamini-Hochberg multiple hypothesis testing correction based on FDR threshold of 0.05.

### Determination of cross resistance / sensitivity

MTT based cell survival assay was performed in either case, whether to determine the cross-resistance of the DAC resistant cell lines towards inhibitors of DNA methyltransferases, histone acetyltransferases, histone methyltransferases, histone deacetylases, and histone demethylases, or to determine their sensitivity towards selective BET bromodomain inhibitors.

The method is primarily based on reduction of yellow colored tetrazolium salt, 3-(4,5-dimethylthiazol-2-yl)-2,5-diphenyltetrazolium bromide (MTT) to insoluble purple colored formazan crystals by NAD(P)H-dependent oxidoreductase enzymes in mitochondria of the viable cell. The intensity of the purple color produced on solubilization of the formazan crystals is directly proportional to the number of viable cells (Meerloo J.V. et al., Methods in Molecular Biology 2011; 731, 237-45).

To determine the IC₅₀ of the epigenetic drugs (Tocris Bioscience, Bristol, United Kingdom), four independent experiments were performed using triplicate wells of 96 well plates. Cells in 80 µL of medium were plated in each of the experimental and the control wells, followed by addition of 20 µL medium with five-fold drug concentration to experimental wells. The wells with medium alone were included alongside as blank for absorbance readings. After 72 h of drug treatment, 10 µL MTT (Sigma-Aldrich) prepared in 1x PBS (5 mg/mL) was added to all wells including blank and control, and incubated until the purple formazan crystals were visible after which 100 µL of detergent (10% SDS, pH: 5.5) was added and the plates were incubated overnight to solubilize the formazan and the cellular material. MTT absorbance was read at 540 nM using Labsystems iEMS Reader MF, and the IC₅₀ values were determined using the Chemorezist software (IMTM, Palacky University, Olomouc, Czech Republic).

Cross resistance was determined as the fold increase, whereas, the sensitivity was determined as the fold decrease in the IC₅₀ of the drugs for the resistant cell lines compared to their genetically identical drug sensitive counterpart.

### Anti-tumor activity of DAC and (+)-JQ1

For *in vivo* validation of HCT116 resistance towards DAC and sensitivity of DAC resistant tumors to (+)-JQ1 treatment, we studied the anti-tumor activity of DAC and (+)-JQ1 in HCT116 parental versus a resistant cell clone R1.4 (HCT116_R_{DAC}). Xenografts were established in 11-12 weeks-old female SCID mice, inoculated with 5 × 10⁶ cells, s.c. on both sides of the chest. After 2 weeks, tumors were palpable (average tumor volume 20 mm³) and mice were assigned into four groups (8 mice/group). Group I: vehicle control for DAC (10:90, DMSO: PBS) and Group II: DAC, 2.5 mg/kg by i.p. injection once a day for 14 days (5 days on, 2 days off), total 10 doses. Group III: vehicle control for (+)-JQ1 (5:95, DMSO: 10% 2-Hydroxypropyl-β-cyclodextrin) and Group IV: (+)-JQ1, 50 mg/kg by i.p. injection once a day for 28 days (5 days on, 2 days off), total 20 doses. Body weights of the animals were measured daily and tumor volume data were collected twice a week. Mice were killed when the body weight decreased >20% of initial weight. All animal work was performed according to approved IACUC protocols.

For determining the anti-tumor activity of the drugs, DAC and (+)-JQ1, tumor volume data for each group were transformed into relative tumor volumes followed by calculation of treatment to control ratio (T/C ratio) for each time point. T/C ratios for day 0 - 21 were further used to compute aAUC for each drug. (Jianrong Wu. Et al., Pharmaceutical statistics 2010; 9, 46-54). aAUC values thus obtained were used to define resistance (for DAC) and sensitivity index [for (+)-JQ1] of HCT116_R_{DAC} in comparison with parental HCT116. Statistical significance of the data was determined by calculating bootstrap p-value, n=10000, one-sided test of H0: T/C ratio = 1, H1: T/C ratio <1 (Jianrong Wu., Journal of Biopharmaceutical Statistics 2010; 20, 954-64). After day 21, statistical significance cannot be measured accurately due to decreased survival of animals in control group.

### Results

The gene and protein expression studies were done at RNA and protein levels respectively. For the gene expression studies, massively parallel signature sequencing (MPSS) was used and the sequences generated from the RNA-Seq experiment were mapped on annotated human reference genome (HG₁₉) followed by quantification of gene and transcript expressions, whereas, for the protein expression studies, stable isotope labelling of amino acids in cell culture (SILAC) was used and the protein expressions were quantified using mass spectrometry.

For reporting the differential expressions, each of the drug resistant cell lines, HCT116-R_{DAC} (R1.1, R1.2, R1.3, R1.4 and R2.1, R2.2) were compared with their genetically identical drug sensitive counterpart or the parental cell line, HCT116. Values are represented as fold changes. Positive values indicate up-regulation and negative values indicate down-regulation. The data is generated from three independent experiments and is statistically significant (p-value <0.05).

| **Gene** | **Change in expression determining resistance** | **Average fold change in DAC resistant cell lines** | | | | | |
|---|---|---|---|---|---|---|---|
| | | **R1.1** | **R1**.**2** | **R1**.**3** | **R1**.**4** | **R2**.**1** | **R2**.**2** |
| ASH1L | increase | | | | | | 0.52 |
| ATAD2 | decrease | | | | | -0.67 | -0.64 |
| BAZ1B | decrease | | | | | -0.40 | |
| BAZ2A | increase | | | 0.33 | 0.33 | | 0.38 |
| BAZ2B | decrease | | | | -0.92 | | |
| BRD1 | decrease | -0.48 | -0.52 | | | | |
| BRD2 | increase | | 0.31 | 0.02 | 0.03 | 0.04 | 0.19 |
| BRD3 | increase | 0.36 | 0.43 | 1.02 | 0.43 | 0.76 | 1.07 |
| BRD4 | decrease | -0.46 | 0.02 | -0.33 | -0.51 | -0.23 | 0.04 |
| BRD7 | decrease | | | | | | -0.55 |
| BRD8 | decrease | -0.44 | -0.35 | | -0.47 | -0.67 | -0.50 |
| BRWD1 | increase | 0.66 | | | | | |
| CECR2 | increase | | | | | | 0.73 |
| CREBBP | increase | | | 0.35 | 0.40 | | |
| EP300 | increase | | | | | | 0.35 |
| KAT2A | increase | 0.37 | | 0.47 | 0.40 | 0.43 | |
| KAT2B | increase | | | | | | 0.62 |
| KMT2A | increase | | 0.45 | | | | |
| SMARCA2 | increase | 0.71 | 0.73 | 0.69 | 0.98 | 0.76 | 1.17 |
| SP100 | increase | 0.77 | 0.59 | | 0.99 | 0.51 | 1.22 |
| SP110 | increase | 0.85 | 0.58 | | 1.29 | | 1.19 |
| TRIM66 | decrease | | | -0.60 | -0.55 | | |
| ZMYND8 | decrease | | | | | -0.50 | -0.50 |
| ZMYND11 | decrease | | -0.55 | | | -0.43 | |

Cross-resistance towards tested epigenetic inhibitors was determined using MTT based cell survival assay and resistance index was calculated as the ratio of IC₅₀ values of the resistant cell lines to their genetically identical drug sensitive counterpart.

The values in the table represents mean IC₅₀ in µM calculated from four independent experiments, each performed in triplicates (S.D, ±0 - ±9.74) and the values in parentheses represents fold changes. The experimental significance was determined using one way Anova with Bonferroni's multiple comparison test (*p<0.05, **p<0.005, ***p<0.0005).

| | **Mean IC₅₀ values in µM (fold changes)** | | | | | | |
|---|---|---|---|---|---|---|---|
| | **HCT116** | **R1.1** | **R1**.**2** | **R1**.**3** | **R1**.**4** | **R2**.**1** | **R2**.**2** |
| DAC | 0.28 | >100 (>357) *** | >100 (>357) *** | >100 (>357) *** | >100 (>357) *** | >100 (>357) *** | >100 (>357) *** |
| AZA | 3.02 | 9.36 (3.10) *** | 11.69 (3.87) *** | 10.57 (3.50) *** | 14.86 (4.92) *** | 12.28 (4.07) *** | 14.47 (4.19) *** |
| Zebularine | 84.16 | 100 | 100 | 100 | 100 | 100 | 100 |
| | | (1.19) *** | (1.19) *** | (1.19) *** | (1.19) *** | (1.19) *** | (1.19) *** |
| Ancardic acid | 120.18 | 128.27 (1.07) | 126.92 (1.06) | 124.80 (1.04) | 122.50 (1.02) | 124.23 (1.03) | 124.71 (1.04) |
| C646 | 33.45 | 45.10 (1.35) | 50.59 (1.51) ** | 47.52 (1.42) * | 51.28 (1.53) ** | 51.02 (1.52) ** | 55 (1.64) *** |
| BIX-01294 | 2.55 | 3 (1.17) | 3.02 (1.19) * | 2.97 (1.16) | 3.05 (1.20) * | 3.40 (1.33) *** | 3.22 (1.26) ** |
| DZNep | 0.82 | 25 (30.34) *** | 25 (30.34) *** | 25 (30.34) *** | 25 (30.34) *** | 25 (30.34) *** | 25 (30.34) *** |
| Romidepsin | 0.0028 | 0.0031 (1.14) | 0.0049 (1.77) | 0.0033 (1.18) | 0.0031 (1.14) | 0.0054 (1.95) * | 0.0073 (2.64) *** |
| Vorinostat | 0.68 | 0.84 (1.24) * | 0.94 (1.39) *** | 0.86 (1.28) * | 0.92 (1.37) *** | 0.75 (1.11) | 0.80 (1.19) |
| GSK J4 | 2.28 | 3.45 (1.52) | 2.95 (1.30) | 3.18 (1.39) | 3.05 (1.34) | 4.81 (2.11) *** | 4.25 (1.87) ** |
| IOX1 | 29.56 | 41.56 (1.41) *** | 63.56 (2.15) *** | 53.18 (1.80) *** | 58.05 (1.96) *** | 50.16 (1.70) *** | 57.02 (1.93) *** |

Sensitivity towards bromodomain inhibitors was determined using MTT based cell survival assay and sensitivity index was calculated as the ratio of IC₅₀ values of the parental cell line to their genetically identical drug resistant counterpart or the resistant cell lines.

The values in the table represents mean IC₅₀ in µM calculated from four independent experiments, each performed in triplicates (S.D, ±0.88 - ±1.09) and the values in parentheses represents fold changes. The experimental significance was determined using one way Anova with Bonferroni's multiple comparison test (*p<0.05, **p<0.005, ***p<0.0005).

| | **Mean IC₅₀ values in µM (fold changes)** | | | | | | |
|---|---|---|---|---|---|---|---|
| | **HCT116** | **R1**.1 | **R1**.**2** | **R1**.**3** | **R1**.**4** | **R2**.**1** | **R2**.**2** |
| **(+)-JQ1** | 3.5 | 0.73 (4.81) *** | 3.17 (1.10) | 0.42 (8.43) *** | 0.78 (4.48) *** | 0.76 (4.60) *** | 3.12 (1.12) |
| **I-BET 151** | 5.23 | 2.68 (1.95) *** | 5.18 (1.01) | 1.60 (3.28) *** | 3.58 (1.46) ** | 3.16 (1.65) *** | 5.40 (0.97) |

Sensitization of DAC resistant cancer cells by bromodomain inhibition was further confirmed in MCF-7 and HL-60 cell lines, parental versus DAC resistant (R_{DAC}). Although the sensitivity of MCF-7_R_{DAC} versus MCF-7 and HL-60_R_{DAC} versus HL-60, towards (+)-JQ1 is not statistically significant, the results apparently show that (+)-JQ1 treatment can overcome high DAC resistance.

| | **Mean IC₅₀ values in µM (fold changes)** | | | |
|---|---|---|---|---|
| | **MCF-7** | **MCF-7_R_{DAC}** | **HL-60** | **HL-60_R_{DAC}** |
| **DAC** | 0.238 | >100 (>421) *** | 0.078 | >100 (>1289) *** |
| **(+)-JQ1** | 0.129 | 0.120 (1.07) | 0.134 | 0.132 (1.01) |

Anti-tumor activity of DAC and (+)-JQ1 was studied in xenografted mouse model of colorectal carcinoma, HCT116 parental versus drug resistant counterpart, HCT116-R_{DAC}. Fig. 1A show the time measurement plots for anti-tumor activity of DAC and (+)-JQ1 compared to vehicle control for each drug, in HCT116 and HCT116-R_{DAC}. Data are relative tumor volume ± SEM. Fig. 1B show the aAUC (T/C ratio) plots comparing the parental HCT116 versus HCT116-R_{DAC}.

### Industrial applicability

Bromodomain containing genes disclosed in the present invention can be used as biomarkers for predicting the clinical response towards the epigenetic therapy targeting aberrant DNA methylation. The varying level of expressions of the genes can be used as a fundament to differentiate between the responders and the non-responders. This provides the accessibility of the method of prediction and personalization of the therapy.

The patients who do not respond to DNA methylation inhibitors and suffer from primary resistance can be quickly eliminated from the ineffective treatment. This will provide the benefit to such patients by escape from the relative side effects that might associate with the drug, redundant cost of therapy, and suggests for other possible treatment protocol in time.

## Claims

1. A method for predicting the sensitivity of a patient suffering from a cancer disease to DNA methylation inhibitor therapy, which comprises determining *in vitro* in cancer cells taken from the patient and comparing with values for parent type of cells
- the level of expression of BRD4, wherein the decrease in expression determines resistance, optionally in combination with one or more or all of the further genes selected from the group comprising:
| **Gene** | **Change in expression determining resistance** |
|---|---|
| ASH1L | increase |
| ATAD2 | decrease |
| BAZ1B | decrease |
| BAZ2A | increase |
| BAZ2B | decrease |
| BRD1 | decrease |
| BRD2 | increase |
| BRD3 | increase |
| BRD7 | decrease |
| BRD8 | decrease |
| BRWD1 | increase |
| CECR2 | increase |
| CREBBP | increase |
| EP300 | increase |
| KAT2A | increase |
| KAT2B | increase |
| KMT2A | increase |
| SMARCA2 | increase |
| SP100 | increase |
| SP110 | increase |
| TRIM66 | decrease |
| ZMYND8 | decrease |
| ZMYND11 | decrease |

2. The method according to claim 1, wherein the level of expression of a combination of BRD4 with at least two, three, four, five, six, seven, eight, nine or ten herein listed bromodomain containing genes is determined.

3. The method according to any one of the preceding claims, wherein the cancer cells are derived from a cancer selected from carcinomas, sarcomas, melanomas, lymphomas, and leukemia.

## Patentansprüche

1. Verfahren zur Vorhersage der Sensitivität eines an einer Krebserkrankung leidenden Patienten auf eine DNA-Methylierungsinhibitor-Therapie, **dadurch gekennzeichnet, dass** *in vitro* in Krebszellen aus dem Patienten bestimmt und mit Werten für den Ausgangstyp der Zellen verglichen werden
- das Expressionsniveau von BRD4, wobei die Verminderung der Expression Resistenz bestimmt, gegebenenfalls in Kombination mit einem oder mehreren oder allen der weiteren Gene ausgewählt aus der Gruppe umfassend:
| **Gen** | **Veränderung des Ausdrucks, der den Widerstand bestimmt** |
|---|---|
| ASH1L | Erhöhung |
| ATAD2 | Verminderung |
| BAZ1B | Verminderung |
| BAZ2A | Erhöhung |
| BAZ2B | Verminderung |
| BRD1 | Verminderung |
| BRD2 | Erhöhung |
| BRD3 | Erhöhung |
| BRD7 | Verminderung |
| BRD8 | Verminderung |
| BRWD1 | Erhöhung |
| CECR2 | Erhöhung |
| CREBBP | Erhöhung |
| EP300 | Erhöhung |
| KAT2A | Erhöhung |
| KAT2B | Erhöhung |
| KMT2A | Erhöhung |
| SMARCA2 | Erhöhung |
| SP100 | Erhöhung |
| SP110 | Erhöhung |
| TRIM66 | Verminderung |
| ZMYND8 | Verminderung |
| ZMYND11 | Verminderung |

2. Verfahren nach Anspruch 1, wobei das Expressionsniveau einer Kombination von BRD4 mit mindestens zwei, drei, vier, fünf, sechs, sieben, acht, neun oder zehn hierin aufgeführten Bromodomäne enthaltenden Genen bestimmt wird.

3. Verfahren nach einem der vorhergehenden Ansprüche, wobei die Krebszellen von einem Krebs stammen, der ausgewählt ist aus Karzinomen, Sarkomen, Melanomen, Lymphomen und Leukämie.

## Revendications

1. Procédé de prédiction de la sensibilité d'un patient souffrant d'une maladie cancéreuse au traitement par inhibiteur de méthylation de l'ADN, qui contient la détermination *in vitro* dans des cellules cancéreuses prélevées chez le patient et la comparation avec des valeurs pour des cellules de type parent
- du niveau d'expression de BRD4, où la diminution de l'expression détermine la résistance, éventuellement en combinaison avec un ou plusieurs ou tous les autres gènes choisis dans le groupe comprenant:
| **Gène** | **Changement d'expression déterminant la résistance** |
|---|---|
| ASH1L | augmentation |
| ATAD2 | diminution |
| BAZ1B | diminution |
| BAZ2A | augmentation |
| BAZ2B | diminution |
| BRD1 | diminution |
| BRD2 | augmentation |
| BRD3 | augmentation |
| BRD7 | diminution |
| BRD8 | diminution |
| BRWD1 | augmentation |
| CECR2 | augmentation |
| CREBBP | augmentation |
| EP300 | augmentation |
| KAT2A | augmentation |
| KAT2B | augmentation |
| KMT2A | augmentation |
| SMARCA2 | augmentation |
| SP100 | augmentation |
| SP110 | augmentation |
| TRIM66 | diminution |
| ZMYND8 | diminution |
| ZMYND11 | diminution |

2. Procédé selon la revendication 1, dans lequel le niveau d'expression d'une combinaison de BRD4 avec au moins deux, trois, quatre, cinq, six, sept, huit, neuf ou dix gènes contenant de la bromodomaine listés ici est déterminé.

3. Procédé selon l'une quelconque des revendications précédentes, dans lequel les cellules cancéreuses sont dérivées d'un cancer choisi parmi les carcinomes, les sarcomes, les mélanomes, les lymphomes et les leucémies.
